# EUROPEAN PATENT APPLICATION

(11) **EP 4 545 113 A1**
(43) Date of publication of application: **30.04.2025**
(21) Application number: 23830799.5
(22) Date of filing: 18.04.2023
(51) Int. Cl.: A61M 1/14, A61M 1/16, A61M 60/113, A61M 60/279, A61M 60/37, A61M 60/585

(54) **BLOOD PURIFICATION DEVICE**

(30) Priority: 27.06.2022 JP 2022102449
(71) Applicant: Nikkiso Co., Ltd., Tokyo 150-6022 (JP)
(72) Inventor: KAWAMURA, Shunsuke, Makinohara-shi, Shizuoka 421-0496 (JP); MAKI, Hideto, Makinohara-shi, Shizuoka 421-0496 (JP)
(74) Representative: von Hirschhausen, Helge
(86) International application number: PCT/JP2023/015483
(87) International publication number: WO 2024/004345

(57) **Abstract**

The present invention is provided with a cassette frame (28) and a cassette frame attachment part (41), wherein: the cassette frame attachment part (41) has locking portions (51, 52, 53) which lock the cassette frame (28) from the front surface side, and a contact-type sensor (54) which presses the cassette frame (28) from the rear surface side; the cassette frame (28) has formed therein lock holes (96) through which the locking portions (51, 52, 53) pass, and a pressure-receiving portion (97) which is pressed by the contact-type sensor (54); and the pressure-receiving portion (97) has formed therein a pressure-receiving surface (97a) which converts a portion of the pressing force from the contact-type sensor (54) into a force in the opposite direction to the direction of locking protrusions (51b 52b, 53b) of the locking portions (51, 52, 53), thereby moving the cassette frame (28) in said opposite direction.

## Description

### TECHNICAL FIELD

The present invention relates to a blood purification device.

### BACKGROUND OF THE INVENTION

There is a blood purification device in which a cassette frame with a pump tube attached is removably attached (see Patent Literature 1). This blood purification device includes a cassette frame (an attaching member) with a pump tube mounted thereon, and plural locking claws that lock the cassette frame from the front surface side. This blood purification device is configured such that attachment of the cassette frame is performed by attaching the cassette frame to the plural locking claws.

### CITATION LIST

### PATENT LITERATURES

Patent Literature 1: Japanese Patent No. 6777721

### SUMMARY OF THE INVENTION

In case of this type of blood purification device, there is a demand for a structure in which the cassette frame is more unlikely to come off during use.

Therefore, it is an object of the invention to provide a blood purification device in which a cassette frame is unlikely to come off during use.

A blood purification device in an embodiment of the invention comprises:
a cassette frame that, when a component of an extracorporeal circulation circuit is attached thereto, forms an integral cassette; and
a cassette frame attachment part to which the cassette frame is removably attached,
wherein the cassette frame attachment part comprises a locking portion that locks the cassette frame from the front surface side, and a pressing portion that presses the cassette frame, which is locked by the locking portion, from the back surface side,
wherein the cassette frame comprises a locking hole through which the locking portion passes, and a pressure-receiving portion that receives pressure from the pressing portion, and
wherein one of the pressing portion and the pressure-receiving portion comprises an inclined surface that comes into contact with the other of the pressing portion and
pressure-receiving portion and converts a part of a pressing force from the pressing portion into a force in an opposite direction to a direction a locking protrusion of the locking portion faces, thereby moving the cassette frame in the opposite direction to the direction the locking protrusion faces.

### Advantageous Effects of the Invention

According to an embodiment of the invention, the cassette frame is unlikely to come off during use.

### BRIEF DESCRIPTION OF DRAWINGS

Fig. 1A is a schematic configuration diagram illustrating a structure of a blood purification device in an embodiment of the present invention.
Fig. 2 is a perspective view showing the blood purification device.
Fig. 3 is a front view showing an area around a cassette frame attachment part.
Fig. 4A is a plan view showing an area around the cassette frame attachment part when locking portions are located in a first locking portion position.
Fig. 4B is a plan view showing the area around the cassette frame attachment part when the locking portions are located in a second locking portion position.
Fig. 5A is a plan view showing the cassette frame and an area around the cassette frame attachment part when the cassette frame is located in a closely-located frame position.
Fig. 5B is a plan view showing the cassette frame and the area around the cassette frame attachment part when the cassette frame is located in a distantly-located frame position.
Fig. 6A is a first explanatory diagram illustrating a method for attaching the cassette frame to the cassette frame attachment part.
Fig. 6B is a second explanatory diagram illustrating the method for attaching the cassette frame to the cassette frame attachment part.
Fig. 6C is a third explanatory diagram illustrating the method for attaching the cassette frame to the cassette frame attachment part.
Fig. 7A is a first explanatory diagram illustrating a method for detaching the cassette frame from the cassette frame attachment part.
Fig. 7B is a second explanatory diagram illustrating the method for detaching the cassette frame from the cassette frame attachment part.
Fig. 7C is a third explanatory diagram illustrating the method for detaching the cassette frame from the cassette frame attachment part.
Fig. 8A is a side view showing a right locking portion.
Fig. 8B is a side view showing a left locking portion.
Fig. 9A is a perspective view showing the cassette frame.
Fig. 9B is a front view showing the cassette frame.
Fig. 10 is a cross-sectional view showing the cassette frame taken along line A-A' in Fig. 9B.
Fig. 11 is a flowchart showing a cassette frame attachment operation.
Fig. 12 is a flowchart showing a cassette frame detachment operation.

### DETAILED DESCRIPTION OF THE INVENTION

A blood purification device in an embodiment of the invention will be described below in reference to the appended drawings. This blood purification device is a medical device that purifies patient's blood using a dialyzer (blood purifier), and is a so-called hemodialysis machine. Particularly, this blood purification device has a cassette frame attachment/detachment structure in which the cassette frame is unlikely to come off when in use and is easily detached when not in use.

### Configuration of Blood purification device

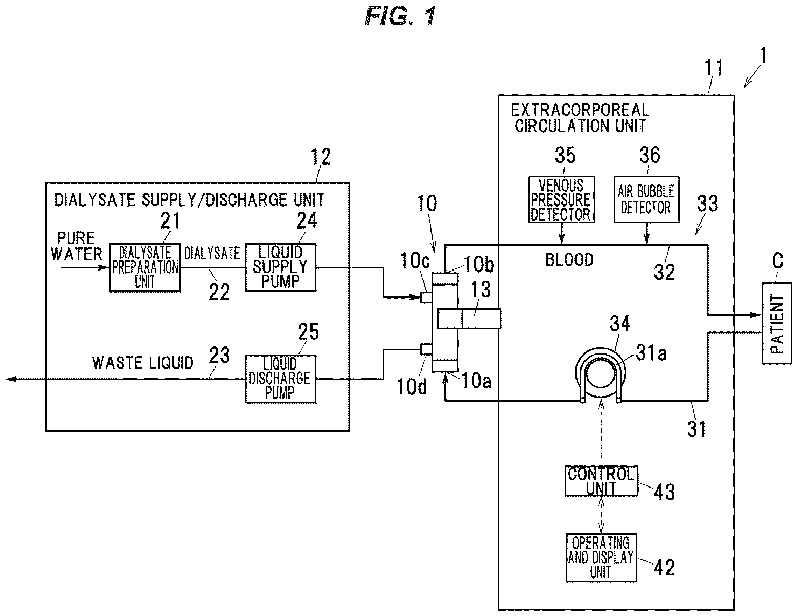

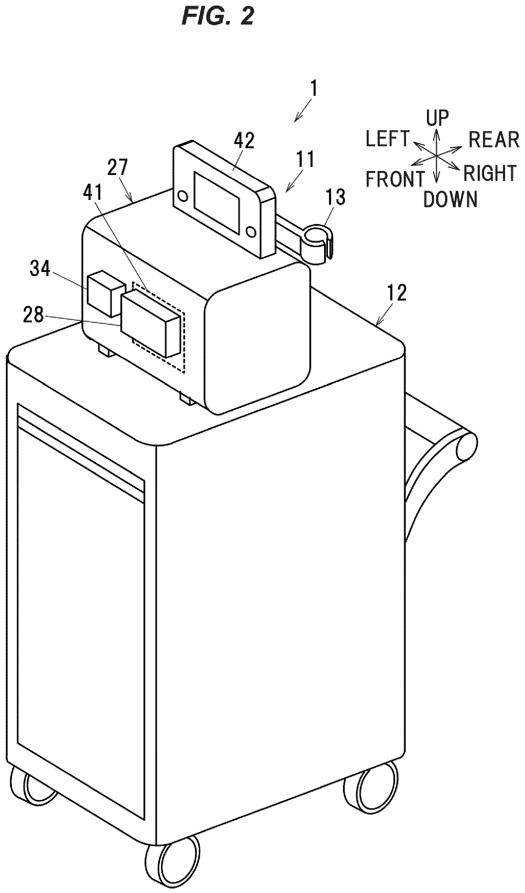

As shown in Fig. 1, a blood purification device 1 includes a dialyzer 10 that purifies blood of a patient C, an extracorporeal circulation unit 11 having an extracorporeal circulation circuit 33 that circulates the blood of the patient C through the dialyzer 10, and a dialysate supply/discharge unit 12 that is connected to the dialyzer 10, supplies dialysate to the dialyzer 10 and also discharges a waste liquid from the dialyzer 10. The dialyzer 10 is removably attached to the extracorporeal circulation unit 11 through a fixing tool 13. In addition, as shown in Fig. 2, the extracorporeal circulation unit 11 and the dialysate supply/discharge unit 12 are configured as separate units and are configured such that the extracorporeal circulation unit 11 can be placed and fixed on the top of the dialysate supply/discharge unit 12.

The dialyzer 10 has a blood purification membrane (a hollow-fiber hemodialysis membrane, a hemodialysis filtration membrane, a flat hemodialysis membrane, or a hemofiltration membrane) thereinside. In addition, as shown in Fig. 1, the dialyzer 10 also has a blood inlet 10a to introduce blood and a blood outlet 10b to discharge the introduced blood, as well as a dialysate inlet 10c to introduce dialysate and a dialysate outlet 10d to discharge the introduced dialysate. In the dialyzer 10, blood is purified by bringing the blood into contact with dialysate through the blood purification membrane.

The dialysate supply/discharge unit 12 has a dialysate preparation unit 21 that refines dialysate, a dialysate supply flow path 22 that is connected to the dialysate inlet 10c of the dialyzer 10 and supplies the dialysate refined by the dialysate preparation unit 21 to the dialyzer 10, and a waste liquid discharge flow path 23 that is connected to the dialysate outlet 10d of the dialyzer 10 and collects and discharges the waste liquid from the dialyzer 10. The dialysate supply/discharge unit 12 also includes a liquid supply pump 24 provided on the dialysate supply flow path 22 to pump the dialysate from the dialysate preparation unit 21, and a liquid discharge pump 25 provided on the waste liquid discharge flow path 23 to pump the waste liquid from the dialyzer 10. By driving the liquid supply pump 24 and the liquid discharge pump 25, the dialysate refined in the dialysate preparation unit 21 is supplied to the dialyzer 10 and, at the same time, the waste liquid from the dialyzer 10 is discharged. The balance of removed water from blood and replaced water in the dialyzer 10 can be adjusted by controlling the liquid supply pump 24 and the liquid discharge 25 and adjusting the flow rate of the liquid supplied to the dialyzer 10 and the flow rate of the liquid discharged from the dialyzer 10. In this regard, the configuration may be such that duplex pumps provided on the dialysate supply flow path 22 and the waste liquid discharge flow path 23 and a water removal pump provided on a bypass flow path bypassing the duplex pump are used instead of the liquid supply pump 24 and the liquid discharge pump 25 which can be independently driven, and liquid supply and liquid discharge to and from the dialyzer 10 are performed by the duplex pumps and the water removal pump.

### Configuration of Extracorporeal circulation unit

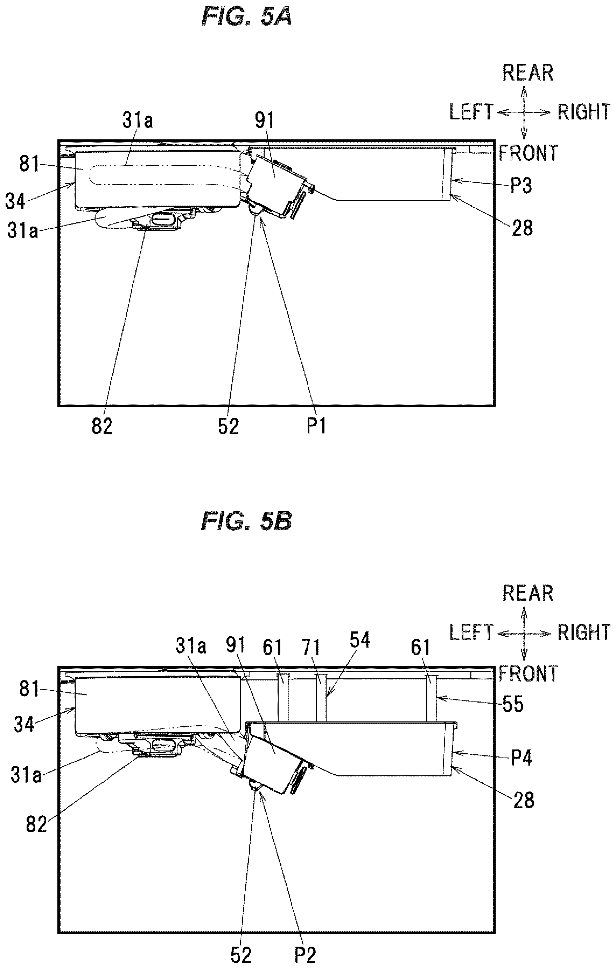

As shown in Figs. 1 and 2, the extracorporeal circulation unit 11 has a unit main body 27. A cassette frame 28 to which consumable components of the extracorporeal circulation circuit 33 are attached is removably attached to the front surface of the unit main body 27. The cassette frame 28 is a frame that, when the consumable components of the extracorporeal circulation circuit 33 are attached thereto, forms an integral cassette (a so-called cassette circuit), i.e., a support frame to attach and detach the consumable components of the extracorporeal circulation circuit 33 as one unit to the unit main body 27. Plural components, which include an arterial blood tube 31 and a venous blood tube 32 (described later) and are not shown in Figs. 5A and 5B except for a pump tube 31a (described later), are attached as the consumable components. Details of the cassette frame 28 will be described later.

### Configuration of Extracorporeal circulation circuit 33

In the extracorporeal circulation unit 11, when the cassette frame 28 is attached to the unit main body 27, the consumable components attached to the cassette frame 28 and components arranged in the unit main body 27 form the extracorporeal circulation circuit 33. As shown in Fig. 1, the extracorporeal circulation circuit 33 has the arterial blood tube 31 that is connected to the blood inlet 10a of the dialyzer 10 and leads the blood collected from a blood vessel of the patient C to the dialyzer 10, and the venous blood tube 32 that is connected to the blood outlet 10b of the dialyzer 10 and returns the blood discharged from the dialyzer 10 to the blood vessel of the patient C. The extracorporeal circulation circuit 33 also has a peristaltic pump 34 arranged on the arterial blood tube 31 to circulate the blood, a venous pressure detector 35 arranged on the venous blood tube 32 to measure pressure of the blood flowing through the extracorporeal circulation circuit 33, and an air bubble detector 36 arranged on the venous blood tube 32 to detect air bubbles in the blood. The arterial blood tube 31 includes the pump tube 31a attached to the peristaltic pump 34.

### Configuration of Unit main body

As shown in Fig, 2, a cassette frame attachment part 41 to which the cassette frame 28 is removably attached, the peristaltic pump 34 mentioned above, and a displacing portion 55 which advances and retracts three locking portions 51, 52, and 53 (described later) as one unit, are arranged on the front surface side of the unit main body 27. The cassette frame 28 is attached to the cassette frame attachment part 41, with its front surface on the front side and its back surface on the rear side. The unit main body 27 also has an operating and display unit 42 arranged at the top and a control unit 43 (see Fig. 1). The operating and display unit 42 is an example of the operation unit. The operating and display unit 42 and the control unit 43 will be described later.

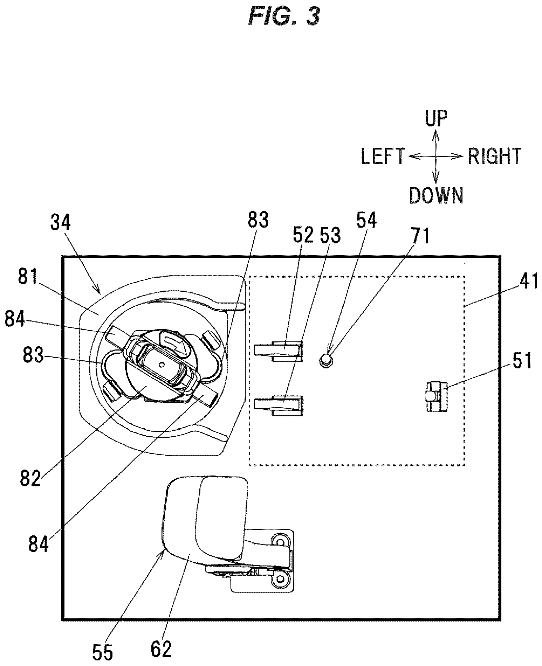

As shown in a dashed line in Fig. 3, the cassette frame attachment part 41 has the three locking portions 51, 52, and 53 that lock the cassette frame 28 from the front surface side (from the forward side), and a contact-type sensor 54 that presses the cassette frame 28, which is locked by the three locking portions 51, 52, and 53, from the back surface side (from the rear side) and also detects attachment of the cassette frame 28.

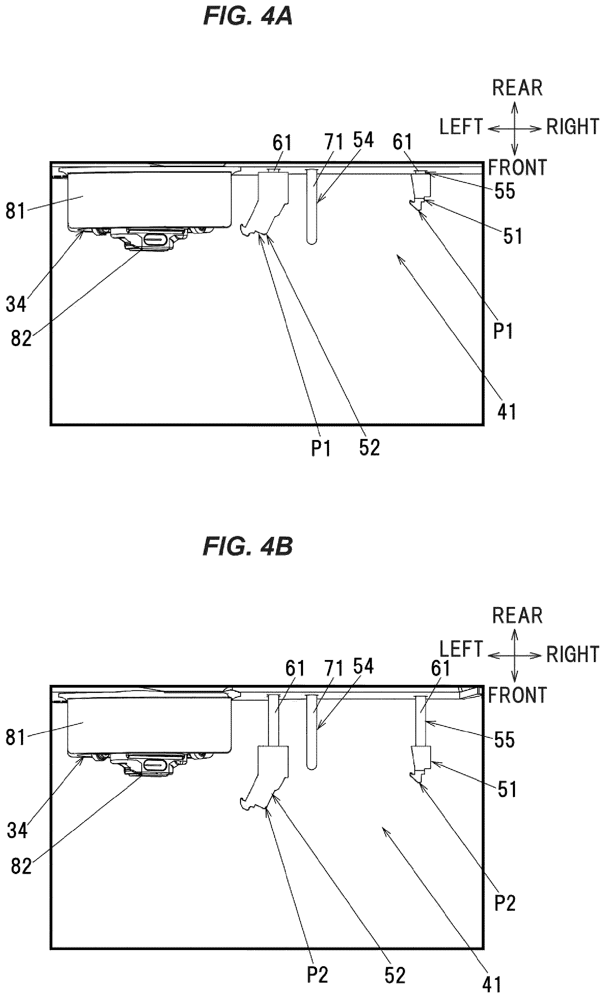

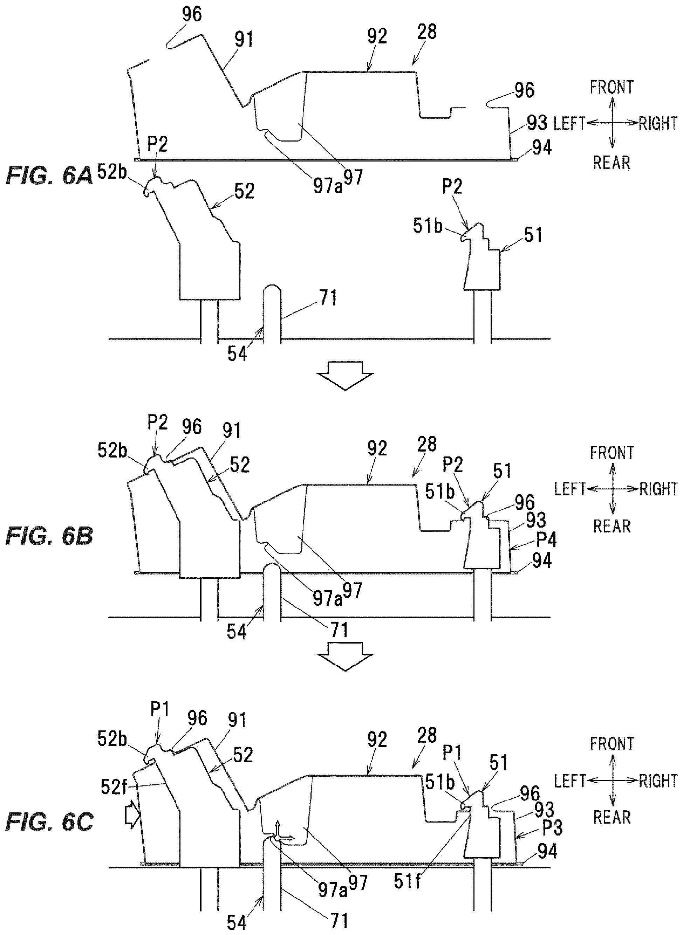

As shown in Figs. 4A and 4B, the displacing portion 55 has, e.g., three support rods 61 respectively supporting the three locking portions 51, 52, and 53, an operating lever 62 operated by a user, and a linkage mechanism (not shown) which moves the three support rods 61 forward and backward in conjunction with the operation performed on the operating lever 62. The displacing portion 55 moves in conjunction with the rotational operation performed on the operating lever 62 and displaces, through the linkage mechanism and the support rods 61, each of the locking portions 51, 52, and 53 between a first locking portion position P1 (see Fig. 4A) and a second locking portion position P2 (see Fig. 4B) which is located forward of the first locking portion position P1. As shown in Figs. 5A and 6C, this first locking portion position P1 is a position where the cassette frame 28 attached to each of the locking portions 51, 52, and 53 is located in a close position close to the unit main body 27 (hereinafter, referred to as the closely-located frame position P3), and the cassette frame 28 is brought into contact with the contact-type sensor 54 by each of the locking portions 51, 52, and 53. On the other hand, as shown in Fig. 5B and 6B, the second locking portion position P2 is a position where the cassette frame 28 attached to each of the locking portions 51, 52 and 53 is located in a distant position at a distance from the unit main body 27 (hereinafter, referred to as the distantly-located frame position P4), and the cassette frame 28 is separated away from the contact-type sensor 54 by each of the locking portions 51, 52, and 53.

As shown in Figs. 6A to 6C, when attaching the cassette frame 28 to the cassette frame attachment part 41, the cassette frame 28 is attached to each of the locking portions 51, 52, and 53 in a state in which each of the locking portions 51, 52, and 53 is located in the second locking portion position P2 (see Fig. 6B). After that, the operating lever 62 is pivotally moved down so that the displacing portion 55 retracts each of the locking portions 51, 52, and 53 to the first locking portion position P1, thereby moving the cassette frame 28 from the distantly-located frame position P4 to the closely-located frame position P3 (see Fig. 6C). This causes the cassette frame 28 to contact and engage with the contact-type sensor 54.

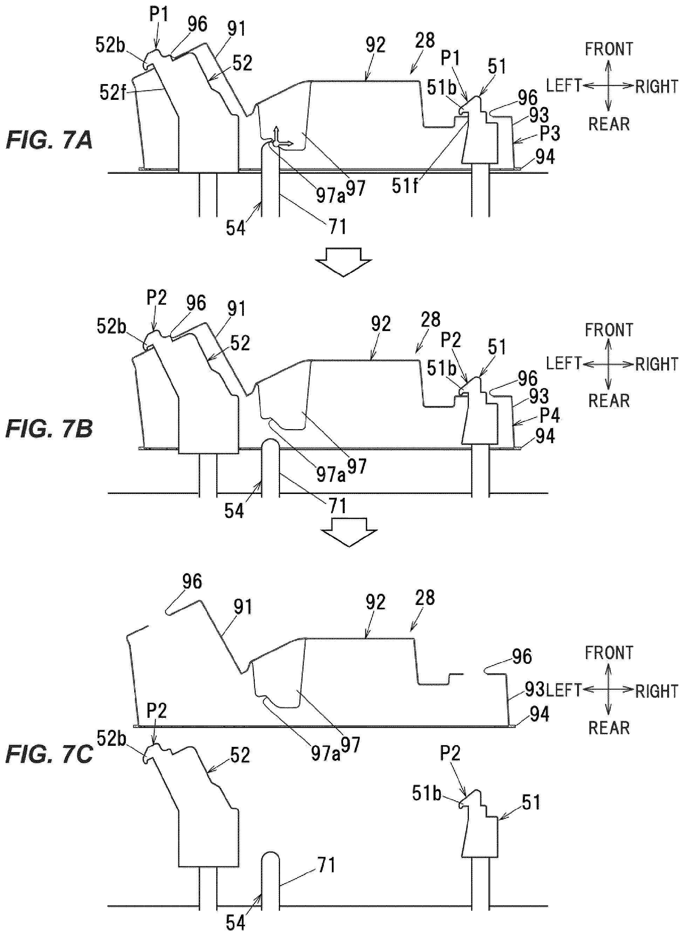

On the other hand, as shown in Figs. 7A to 7C, when detaching the cassette frame 28 from the cassette frame attachment part 41, the operating lever 62 is pivotally moved up so that the displacing portion 55 advances each of the locking portions 51, 52 and 53 to the second locking portion position P2, thereby moving the cassette frame 28 from the closely-located frame position P3 to the distantly-located frame position P4 (see Fig. 7B). This causes the cassette frame 28 to separate away from the contact-type sensor 54. After that, the cassette frame 28 is detached from each of the locking portions 51, 52 and 53 which are located in the second locking portion position P2 (see Fig. 7C).

In the present embodiment, the displacing portion 55 has the three support rods 61, the operating lever 62 and the linkage mechanism, and the user manually displaces each of the locking portions 51, 52, and 53 by operating the operating lever 62. However, an electric actuator may be provided in place of the operating lever 62 to electrically displace each of the locking portions 51, 52, and 53. Furthermore, the three support rods 61 may be integrally formed respectively with the locking portions 51.

As shown in Figs. 4A and 4B, the three locking portions 51, 52, and 53 consist of one right locking portion 51 arranged on the right side and two left locking portions 52 and 53 arranged on the left side.

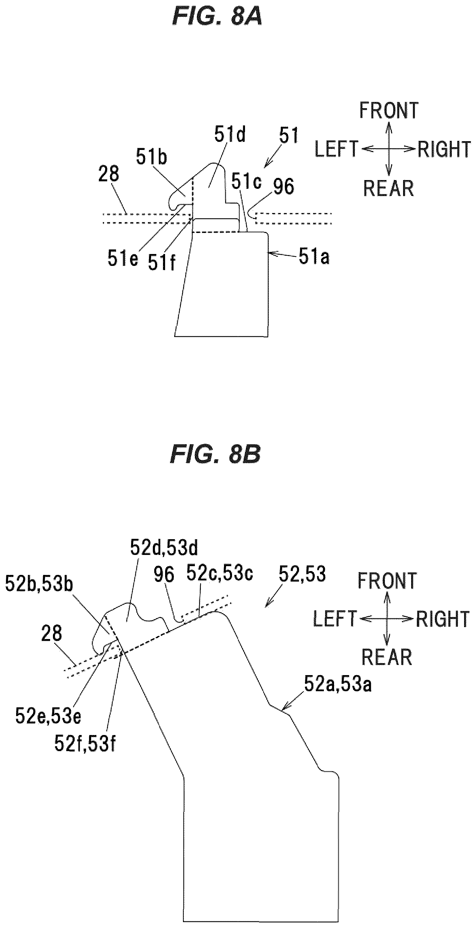

As shown in Fig. 8A, the right locking portion 51 has a base body portion 51a having a tip portion 51d that passes through a locking hole 96 (described later) formed on the cassette frame 28 and engages with the locking hole 96, and a locking protrusion 51b protruding from the tip portion 51d of the base body portion 51a. The locking protrusion 51b of the right locking portion 51 is formed to protrude to the left after passing through the locking hole 96 when viewed from the front (when viewed from the front surface side of the cassette frame 28) and its tip comes into contact with the front surface side of the cassette frame 28 and locks the cassette frame 28. That is, the direction the locking protrusion 51b of the right locking portion 51 faces is leftward. The tip of the locking protrusion 51b is formed in a wedge shape protruding from a bottom surface of a locking protrusion bottom portion 51e toward the front surface side of the cassette frame 28. In the present embodiment, the locking protrusion bottom portion 51e includes the wedge-shaped portion. In this regard, the shape of the locking protrusion 51b only needs to be a shape that can lock the cassette frame 28 from the front surface side, and it may be a claw shape or a simple protrusion shape (a rectangular or mountain shape). The base body portion 51a of the right locking portion 51 also has a contact portion 51c that comes into contact with the back surface side of the cassette frame 28.

As shown in Fig. 8B, the left locking portions 52 and 53 respectively have base body portions 52a and 53a having tip portions 52d and 53d that pass through locking holes 96 (described later) formed on the cassette frame 28 and engage with the locking holes 96, and locking protrusions 52b and 53b protruding from the tip portions 52d and 53d of the base body portions 52a and 53a. The locking protrusions 52b and 53b of the left locking portions 52 and 53 are formed to protrude to the left after passing through the locking holes 96 when viewed from the front (when viewed from the front surface side of the cassette frame 28) and their tips come into contact with the front surface side of the cassette frame 28 and lock the cassette frame 28. That is, the direction the locking protrusions 52b and 53b of the left locking portions 52 and 53 face is leftward. The tips of the locking protrusions 52b and 53b are formed in a wedge shape protruding from bottom surfaces of locking protrusion bottom portions 52e and 53e toward the front surface side of the cassette frame 28. In the present embodiment, the locking protrusion bottom portions 52e and 53e include the wedge-shaped portion. In this regard, the shape of the locking protrusions 52b and 53b only needs to be a shape that can lock the cassette frame 28 from the front surface side, and it may be a claw shape or a simple protrusion shape (a rectangular or mountain shape). The base body portions 52a and 53a of the left locking portions 52 and 53 also respectively have contact portions 52c and 53c that come into contact with the back surface side of the cassette frame 28.

As described above, the right locking portion 51 and the two left locking portions 52 and 53 have the locking protrusions 51b, 52b, and 53b all facing the same direction, i.e., all protrude to the left after passing through the locking holes 96 of the cassette frame 28. Thus, the detachment direction of the cassette frame 28 from the right locking portion 51 and that from the two left locking portions 52 and 53 are both leftward, allowing the detachment of the cassette frame 28 from the right locking portion 51 and the detachment of the cassette frame 28 from the two left locking portions 52 and 53 to be performed simultaneously. Likewise, the attachment direction of the cassette frame 28 to the right locking portion 51 and that to the two left locking portions 52 and 53 are both rightward, allowing the attachment of the cassette frame 28 to the right locking portion 51 and the attachment of the cassette frame 28 to the two left locking portions 52 and 53 to be performed simultaneously.

In addition, since the contact portions 51c, 52c, and 53c, which come into contact with the back surface side of the cassette frame 28 are respectively formed on the base body portions 51a, 52a, and 53a of the locking portions 51, 52, and 53, the cassette frame 28 can be lightly held by the locking of the cassette frame 28 by the locking protrusions 51b, 52b, and 53b and the contact of the contact portions 51c, 52c, and 53c with the cassette frame 28 to the extent that does not hinder the detachment. This makes it possible to prevent the cassette frame 28 from unintentionally falling out of the locking portions 51, 52, and 53 when the cassette frame 28 is located in the distantly-located frame position P4.

As shown in Figs. 3, 4A and 4B, the contact-type sensor 54 has a rod-shaped contact 71 that comes in contact with the back surface side of the cassette frame 28, a bias spring (not shown) that biases the contact 71 forward, and a sensing portion (not shown) that detects retraction of the contact 71. When the cassette frame 28 is displaced from the distantly-located frame position P4 to the closely-located frame position P3, the contact 71 comes into contact with the back surface side (a pressure-receiving portion 97 described later) of the cassette frame 28 and the contact 71 retracts against the biasing force of the biasing spring. When the contact 71 retracts, the retraction of the contact 71 is detected by the sensing portion, and the attachment of the cassette frame 28 is detected based on this detection. Meanwhile, in the state in which the cassette frame 28 has been displaced to the closely-located frame position P3, the contact 71 presses the cassette frame 28 from the back surface side by the biasing force (the restoring force) of the biasing spring. In this manner, in the present embodiment, the contact-type sensor 54 constitutes the pressing portion by which the cassette frame 28 locked from the front surface side by the three locking portions 51, 52, and 53 is pressed from the back surface side.

As shown in Fig. 3, the peristaltic pump 34 is to pump blood by applying pressure to blood (fluid) in the pump tube 31a, and has a stator 81 with an attachment recess, a rotor 82 arranged in the attachment recess and rotated by a motor (not shown in the drawing), plural rollers 83 attached to the rotor 82, and plural guide pins 84 formed on the circumferential surface of the rotor 82 to guide the pump tube 31a. The plural guide pins 84 include plural pairs of upper and lower guide pins 84 arranged in the circumferential direction of the rotor 82. Then, by rotating the rotor 82 with the pump tube 31a introduced between the paired upper and lower guide pins 84, the pump tube 31a is squeezed by the rollers 83 and the blood is pumped. Hereinafter, the space between the paired upper and lower guide pins 84 will be referred to as the interior of the peristaltic pump 34, and the outer side of the paired upper and lower guide pins 84 will be referred to as the exterior of the peristaltic pump 34.

The peristaltic pump 34 also has a function of automatically attaching and detaching the pump tube 31a (an autoloading function). In other words, the peristaltic pump 34 has a loading function of introducing and attaching the pump tube 31a in the interior thereof by driving the rotor 82, and an unloading function of ejecting the pump tube 31a to the exterior and detaching it by driving the rotor 82. In loading, by driving the rotor 82 from a loading preparation position, which is a predetermined rotational position, in the state in which the pump tube 31a routed along the gap between the stator 81 and the rotor 82 is pressed from the front side, the pump tube 31a is wound and introduced into the peristaltic pump 34 by the guide pins 84 (see the phantom line in Fig. 5A). On the other hand, in unloading, by driving the rotor 82 from an unloading preparation position, which is a predetermined rotational position, in the state in which the pump tube 31a is pulled forward, the pump tube 31a is unwound and ejected to the exterior of the peristaltic pump 34 by the guide pins 84 (see the phantom line in Fig. 5B).

### Configuration of Cassette frame

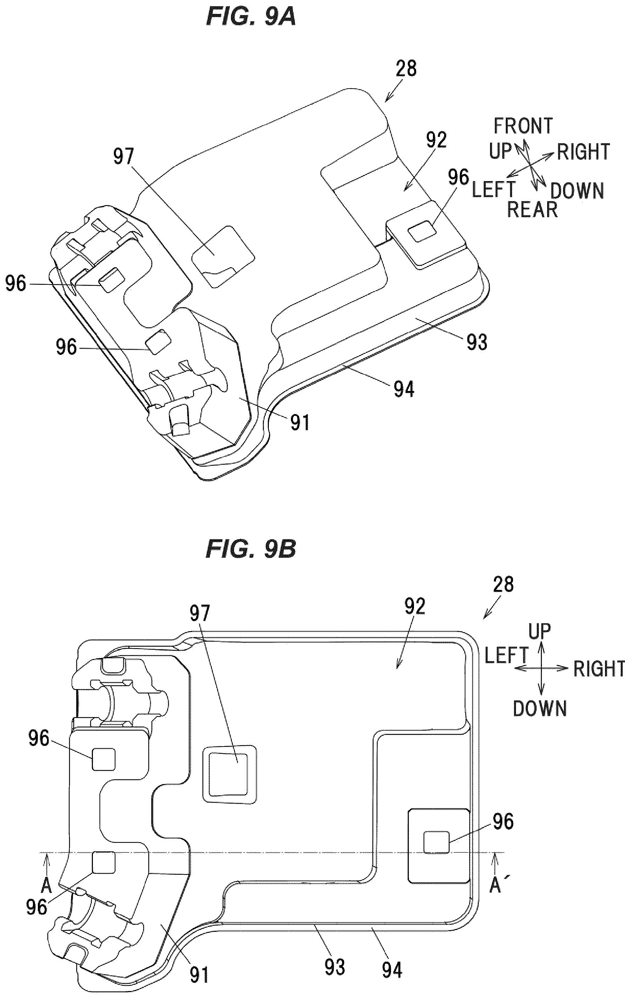

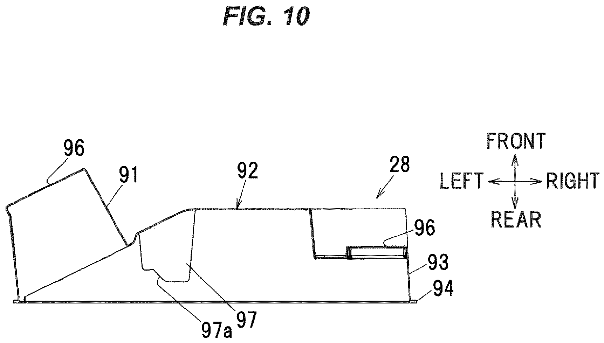

Next, the cassette frame 28 will be described in reference to Figs. 9A, 9B and 10. As shown in Figs. 9 and 10, the cassette frame 28 is, e.g., a molded resin article formed in a block shape with an opening on a rear surface, and integrally has a front wall 92 having attachment portions for various components including a pump tube attachment portion 91, a side wall 93 extending rearward from a peripheral edge of the front wall 92, and a flange portion 94 formed to protrude outward from a rear edge of the side wall 93. The flange portion 94 may be omitted.

The cassette frame 28 also has three locking holes 96 formed on the pump tube attachment portion 91 and the front wall 92 at positions corresponding to the three locking portions 51, 52, and 53. The cassette frame 28 further has the pressure-receiving portion 97 formed substantially in the center of the front wall 92 to receive pressure from the contact-type sensor 54. The location of the pressure-receiving portion 97 is not limited to the substantially central position of the cassette frame 28, as long as it can receive the pressure from the contact-type sensor 54.

The three locking holes 96 are through-holes that the three locking portions 51, 52, and 53 respectively pass through and engage with. In particular, in the state in which the locking portions 51, 52, and 53 respectively pass through the locking holes 96, inner edges of the locking holes 96 respectively come into contact with vertical side surfaces of the tip portions 51d, 52d, and 53d of the base body portions 51a, 52a, and 53a of the corresponding locking portions 51, 52, and 53, and the cassette frame 28 is locked in the vertical direction by each of the locking portions 51, 52, and 53. In addition, as will be described in detail later, in the state in which the locking portions 51, 52, and 53 respectively pass through the locking holes 96, the inner edges of the locking holes 96 are respectively pressed against locking protrusion-side side surfaces 51f, 52f, and 53f (see Figs. 8A to 8C) of the locking portions 51, 52, and 53 by a pressing force from the contact-type sensor 54, and the cassette frame 28 is thereby locked in the left-right direction by each of the locking portions 51, 52, and 53. The size of the locking hole 96 is not limited to the size in the present embodiment as long as the locking portions 51, 52, and 53 can be inserted therethrough, but its size is preferably such that pushing the locking portions 51, 52, and 53 into the locking holes 96 achieves insertion of the locking portions 51, 52, and 53 into the locking holes 96. In addition, the shape of the locking hole 96 is not limited to the rectangular shape as in the present embodiment, and may be a circular shape or a polygonal shape other than the rectangular shape as long as it is a shape allowing for locking by the locking portions 51, 52, and 53.

As shown in Fig. 10, the pressure-receiving portion 97 is formed in a raised shape protruding rearward (toward the back side), and has a pressure-receiving surface 97a formed at its tip with which the contact 71 of the contact-type sensor 54 comes into contact. The pressure-receiving surface 97a is an inclined surface that gradually extends rearward from the left side to the right side. Therefore, a part of the pressing force from the contact-type sensor 54 is converted into a rightward force by the pressure-receiving surface 97a, as shown in Fig. 6C. This allows the pressing force from the contact-type sensor 54 to move the cassette frame 28 to the right and to press the inner edges of the locking holes 96 of the cassette frame 28 respectively against the locking protrusion-side side surfaces 51f, 52f, and 53f the locking portions 51, 52, and 53. The direction of the locking protrusions 51b, 52b, and 53b of the three locking portions 51, 52, and 53 face is leftward and the direction of detaching the cassette frame 28 from each of the locking portions 51, 52, and 53 is also leftward, whereas a part of the pressing force from the contact-type sensor 54 is converted by the pressure-receiving surface 97a into a force in an opposite direction (rightward) to the facing direction of the locking protrusions 51b, 52b, and 53b of the locking portions 51, 52, and 53 and the detachment direction and causes movement in this opposite direction. This makes the cassette frame 28 unlikely to come off the locking portions 51, 52, and 53 when the blood purification device 1 is in use.

In the present embodiment, the pressure-receiving portion 97 is formed in a raised shape. However, the pressure-receiving portion 97 may not be formed in a raised shape as long as it has the pressure-receiving surface 97a. For example, the pressure-receiving portion 97 may be formed in a recessed shape.

In addition, the pressure-receiving surface 97a is straight and flat in the present embodiment, but it is not limited thereto as long as a part of the pressing force from the contact-type sensor 54 can be converted into a rightward force. For example, the pressure-receiving surface 97a may be a rounded surface which is curved.

In addition, in the present embodiment, the pressing force from the contact-type sensor 54 moves the cassette frame 28 to the right and presses the inner edges of the locking holes 96 of the cassette frame 28 against the locking protrusion-side side surfaces 51f, 52f, and 53f of the locking portions 51, 52, and 53. However, the configuration may be such that the pressing force from the contact-type sensor 54 moves the cassette frame 28 to the right to the extent that the inner edges of the locking holes 96 do not come into contact with the locking protrusion-side side surfaces 51f, 52f, and 53f.

The pump tube attachment portion 91 supports both ends of the pump tube 31a in a state in which the pump tube 31a is in a "U" shape along the gap between the stator 81 and the rotor 82 of the peristaltic pump 34. The pump tube attachment portion 91 supports the both ends of the pump tube 31a so that the pump tube 31a outside the peristaltic pump 34 is pressed from the front side against the peristaltic pump 34 when the cassette frame 28 is located in the closely-located frame position P3 (see Fig. 5A), and the pump tube 31a inside the peristaltic pump 34 is pulled toward the front side when the cassette frame 28 is located in the distantly-located frame position P4 (see Fig. 5B). Thus, the peristaltic pump 34 can be loaded in the state in which the cassette frame 28 is located in the closely-located frame position P3 and the peristaltic pump 34 can be unloaded in the state in which the cassette frame 28 is located in the distantly-located frame position P4.

### Configurations of Operating and display unit and Control unit

Next, the operating and display unit 42 and the control unit 43 will be described in reference to Fig. 1. As shown in Fig. 1, the operating and display unit 42 is composed of a monitor having operation buttons and a touch panel, displays various information, and receives various operations from the user. In the present embodiment, the operating and display unit 42 receives self-diagnostic operation, loading operation, unloading preparation operation, and unloading operation.

The control unit 43 is realized by appropriately combining an arithmetic element such as CPU, a memory, software, interface and a communication unit, etc., and performs various controls of the blood purification device 1. In the present embodiment, the control unit 43 controls the peristaltic pump 34 to execute self-diagnostic action, loading preparation action, loading action, unloading preparation action, and unloading action.

The self-diagnostic action is an action to diagnose the peristaltic pump 34 (to determine whether there is an abnormality) by performing test drive of the peristaltic pump 34 (forward drive, reverse drive, and stop). The loading preparation action is an action to rotate the rotor 82 to the loading preparation position. The loading action is an action to introduce the pump tube 31a into the peristaltic pump 34 by driving the peristaltic pump 34 in the state in which the rotor 82 is located in the loading preparation position. The unloading preparation action is an action to rotate the rotor 82 to the unloading preparation position. The unloading action is an action to eject the pump tube 31a out of the peristaltic pump 34 by driving the peristaltic pump 34 in the state in which the rotor 82 is located in the unloading preparation position.

Particularly, when receiving the self-diagnostic operation through the operating and display unit 42, the control unit 43 executes the loading preparation action in addition to the self-diagnostic action. Executing the self-diagnostic action and the loading preparation action by a single operation in this manner can reduce the user's time and effort for operation.

### Description of Cassette frame attachment operation

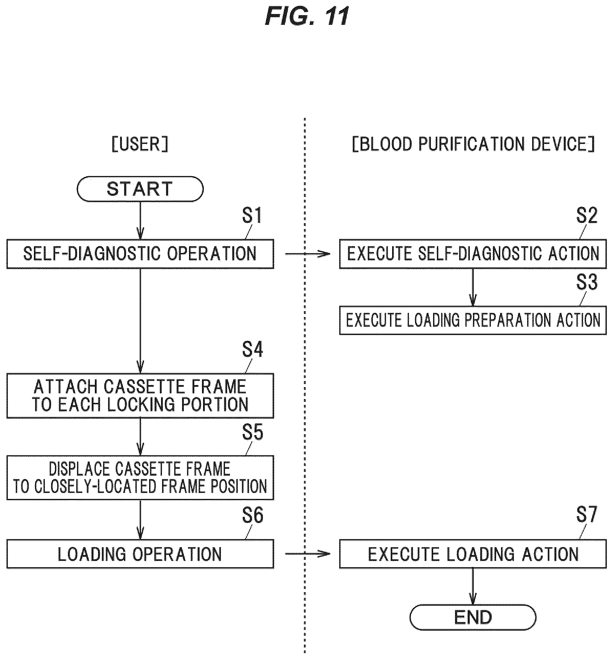

Next, the cassette frame attachment operation will be described in reference to Figs. 6A to 6C and 11. The cassette frame attachment operation is an operation to attach the cassette frame 28 to the cassette frame attachment part 41, and is performed in a state in which each of the locking portions 51, 52, and 53 is located in the second locking portion position P2 (see Fig. 6A). As shown in Fig. 11, first, the user performs a self-diagnostic operation on the operating and display unit 42 (S1). Then, the control unit 43 receives the self-diagnostic operation through the operating and display unit 42, and executes the self-diagnostic action by controlling the peristaltic pump 34 (S2). After that, the peristaltic pump 34 is controlled to execute the loading preparation action (S3). That is, after diagnosis of the peristaltic pump 34 by performing the test drive of the peristaltic pump 34, the rotor 82 is rotated to the loading preparation position.

After executing the self-diagnostic action and the loading preparation action, the user attaches the cassette frame 28 to the locking portions 51, 52, and 53 (S4) (see Fig. 6B). The operating lever 62 is then operated so that the cassette frame 28 is displaced by the displacing unit 55 from the distantly-located frame position P4 to the closely-located frame position P3 (S5) (see Fig. 6C). This displacement causes the contact 71 of the contact-type sensor 54 to come into contact with the pressure-receiving portion 97 of the cassette frame 28, and the pressure-receiving surface 97a of the pressure-receiving portion 97 causes the contact-type sensor 54 to push the cassette frame 28 toward the front side and toward the right side. In addition, the pump tube 31a attached to the cassette frame 28 is pressed from the front side against the peristaltic pump 34.

After the displacement of the cassette frame 28 to the closely-located frame position P3, the user performs a loading operation on the operating and display unit 42 (S6). Then, the control unit 43 receives the loading operation through the operating and display unit 42, and executes the loading action by controlling the peristaltic pump 34 (S7). The pump tube 31a is thereby introduced into the peristaltic pump 34. The cassette frame attachment operation is thereby ended.

### Description of Cassette frame detachment operation

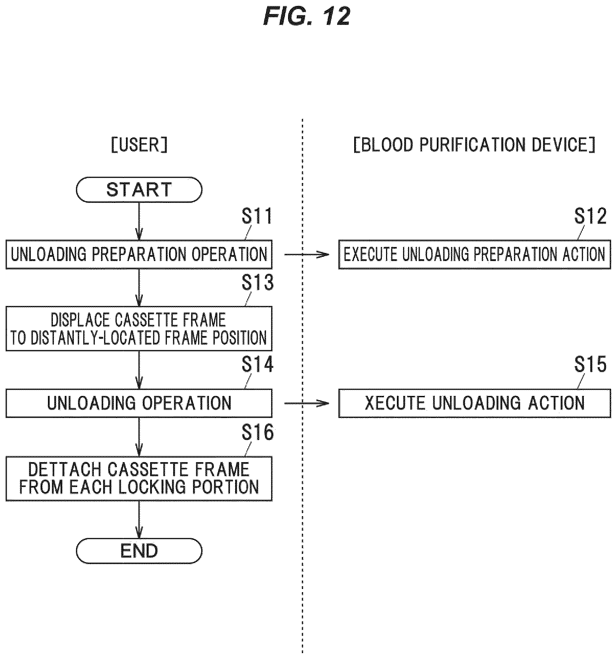

Next, the cassette frame detachment operation will be described in reference to Figs. 7A to 7C and 12. The cassette frame detachment operation is an operation to detach the cassette frame 28 from the cassette frame attachment part 41, and is performed in a state in which each of the locking portions 51, 52, and 53 is located in the first locking portion position P1 and the cassette frame 28 is located in the closely-located frame position P3 (see Fig. 7A). As shown in Fig. 12, first, the user performs an unloading preparation operation on the operating and display unit 42 (S11). Then, the control unit 43 receives the unloading preparation operation through the operating and display unit 42, and executes the unloading preparation action by controlling the peristaltic pump 34 (S12). That is, the rotor 82 is rotated to the unloading preparation position.

After executing the unloading preparation action, the user operates the operating lever 62 so that the cassette frame 28 is displaced by the displacing unit 55 from the closely-located frame position P3 to the distantly-located frame position P4 (S13) (see Fig. 7B). This displacement causes the cassette frame 28 to be separated from the contact-type sensor 54. It also causes the pump tube 31a attached to the cassette frame 28 to be pulled forward.

After the displacement of the cassette frame 28 to the distantly-located frame position P4, the user performs an unloading operation on the operating and display unit 42 (S14). Then, the control unit 43 receives the unloading operation through the operating and display unit 42, and executes the unloading action by controlling the peristaltic pump 34 (S15). The pump tube 31a is thereby ejected out of the peristaltic pump 34. After that, the user detaches the cassette frame 28 from the locking portions 51, 52, and 53 (S16) (see Fig. 7C). The cassette frame detachment operation is thereby ended.

### Functions and Effects of the embodiment

As described above, in the configuration of the embodiment described above, the cassette frame 28 is unlikely to come off during use and also the cassette frame 28 can be detached easily. That is, since the pressure-receiving surface 97a which converts a part of the pressing force from the contact-type sensor 54 into a force in the opposite direction to the facing direction of the locking protrusions 51b, 52b, and 53b of the locking portions 51, 52, and 53 is provided on the pressure-receiving portion 97, the cassette frame 28 is pressed in an opposite direction to the direction of detaching each of the locking portions 51, 52, and 53 and the cassette frame 28 moves in this opposite direction, which makes the cassette frame 28 unlikely to come off when the blood purification device 1 is in use. This suppresses unintentional detachment of the cassette frame 28 when the blood purification device 1 is in use. In addition, since the locking protrusions 51b, 52b, and 53b of the three locking portions 51, 52, and 53 face the same direction, it is possible to detach the cassette frame 28 from the locking portions 51, 52, and 53 at the same time, and it is thus possible to easily detach the cassette frame 28. This reduces the time and effort required to detach the cassette frame 28.

In addition, in the configuration of the embodiment described above, since the displacing portion 55 which moves the cassette frame 28 between the closely-located frame position P3 and the distantly-located frame position P4 is provided, it is possible to bring the cassette frame 28 in contact with the contact-type sensor 54 by moving the cassette frame 28 to the closely-located frame position P3 when in use and to separate the cassette frame 28 away from the contact-type sensor 54 by moving the cassette frame 28 to the distantly-located frame position P4 when not in use. Since this makes it possible to detach the cassette frame 28 without receiving pressure from the contact-type sensor 54 when not in use, it is possible to realize the blood purification device 1 in which the cassette frame 28 is more unlikely to come off when in use and the cassette frame 28 is detached more easily when not in use.

In addition, in the configuration of the embodiment described above, since the pressing portion to press the cassette frame 28, which is locked by the locking portions 51, 52, and 53, from the back surface side is composed of the contact-type sensor 54, there is no need to provide a pressing portion separately from the contact-type sensor 54, allowing the cassette frame attachment part 41 and the blood purification device 1 to have a simple configuration.

In addition, in the configuration of the embodiment described above, since the locking portions 51, 52, and 53 have the contact portions 51c, 52c, and 53c that come into contact with the back surface side of the cassette frame 28, the cassette frame 28 can be lightly held by the locking of the cassette frame 28 by the locking protrusions 51b, 52b, and 53b and the contact of the contact portions 51c, 52c, and 53c with the cassette frame 28 to the extent that does not hinder the detachment. This makes it possible to prevent the cassette frame 28 from unintentionally falling out of the locking portions 51, 52, and 53 when the cassette frame 28 is located in the distantly-located frame position P4. The contact portions 51c, 52c, and 53c also serve to disengage the cassette frame 28 from the contact-type sensor 54 by coming into contact with the back surface side of the cassette frame 28 when each of the locking portions 51, 52, and 53 is displaced from the first locking portion position P1 to the second locking portion position P2.

In addition, in the configuration of the embodiment described above, since the peristaltic pump 34 has an autoloading function, it is possible to attach the cassette frame 28 easily.

In addition, in the configuration of the embodiment described above, since the loading preparation action is executed in addition to the self-diagnostic action when receiving the self-diagnostic operation through the operating and display unit 42, the self-diagnostic action and the loading preparation action can be executed by a single operation and it is thereby possible to reduce the user's time and effort for operation.

### Modifications

Although the embodiment of the invention has been described, the invention according to claims is not to be limited to the embodiment described above. Further, please note that not all combinations of the features described in the embodiment are necessary to solve the problem of the invention.

For example, the inclined surface which converts a part of the pressing force from contact-type sensor 54 into a force in the opposite direction to the facing direction of locking protrusions 51b, 52b, and 53b of the locking portions 51, 52, and 53 is provided on the pressure-receiving portion 97 in the embodiment described above, but the inclined surface may be provided on the contact-type sensor 54. That is, the inclined surface may be provided at a tip portion of the contact 71 of the contact-type sensor 54. Moreover, the inclined surface may be provided on both the pressure-receiving portion 97 and the contact-type sensor 54.

In addition, although the three locking portions 51, 52, and 53 are provided in the embodiment described above, it is not limited thereto. That is, it may be configured to have only one or two of the locking portions 51, 52, and 53, or to have not less than four. When there is only one locking portion 51, 52 or 53, the effect of easily detaching the cassette frame 28 can be exerted without configuring the locking protrusions 51b, 52b, and 53b to face the same direction.

In addition, although the pressing portion which presses the cassette frame 28 from the back surface side is composed of the contact-type sensor 54 in the embodiment described above, it is not limited thereto. That is, the pressing portion may be provided separately from the contact-type sensor 54.

In addition, the invention is applied to the blood purification device 1 in the embodiment described above, but the invention may be applied to other medical devices (e.g., various therapy equipment or various testing devices).

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.
(1) A blood purification device 1 comprising: a cassette frame 28 that, when a component of an extracorporeal circulation circuit 33 is attached thereto, forms an integral cassette; and a cassette frame attachment part 41 to which the cassette frame 28 is removably attached, wherein the cassette frame attachment part 41 comprises a locking portion 51, 52, 53 that locks the cassette frame 28 from the front surface side, and a pressing portion 54 that presses the cassette frame 28, which is locked by the locking portion 51, 52, 53, from the back surface side, wherein the cassette frame 28 comprises a locking hole 96 through which the locking portion 51, 52, 53 passes, and a pressure-receiving portion 97 that receives pressure from the pressing portion 54, and wherein one of the pressing portion 54 and the pressure-receiving portion 97 comprises an inclined surface 97a that comes into contact with the other of the pressing portion 54 and pressure-receiving portion 97 and converts a part of a pressing force from the pressing portion 54 into a force in an opposite direction to a direction a locking protrusion 51b, 52b, 53b of the locking portion 51, 52, 53 faces, thereby moving the cassette frame 28 in the opposite direction to the direction the locking protrusion 51b, 52b, 53b faces. This makes the cassette frame unlikely to come off during use.
(2) The blood purification device 1 as defined by (1), wherein the cassette frame attachment part 41 comprises a plurality of the locking portions 51, 52, 53, wherein the locking protrusions 51b, 52b, 53b of the plurality of locking portions 51, 52, 53 face the same direction, and wherein the inclined surface 97a converts a part of the pressing force from the pressing portion 54 into a force in an opposite direction to the direction the locking protrusions 51b, 52b, 53b of the plurality of locking portions 51, 52, 53 face. This makes it possible to hold the cassette frame more stably. In addition, since arranging the locking protrusions of the plural locking portions to face the same direction allows the cassette frame to be detached from each of the locking portions at the same time, it is possible to detach the cassette frame easily. This reduces the time and effort required to detach the cassette frame.
(3) The blood purification device 1 as defined by (1) or (2), wherein the cassette frame attachment part 41 further comprises a displacing portion 55 that displaces the locking portion 51, 52, 53 between a first locking portion position P1, at which the cassette frame 28 comes into contact with the pressing portion 54, and a second locking portion position P2, at which the cassette frame 28 is separated from the pressing portion 54.
   It is thereby possible to realize the blood purification device in which the cassette frame is more unlikely to come off when in use and the cassette frame is detached more easily when not in use.
(4) The blood purification device 1 as defined by any one of (1) or (3), wherein the pressing portion 54 comprises a contact-type sensor 54 that comes into contact with the pressure-receiving portion 97 to detect attachment of the cassette frame 28. This allows the cassette frame attachment part and the blood purification device to have a simple configuration.
(5) The blood purification device 1 as defined by any one of (1) to (4), wherein the locking portion 51, 52, 53 comprises a contact portion 51c, 52c, 53c that comes into contact with the back surface side of the cassette frame 28.
   It is thereby possible to prevent the cassette frame from unintentionally falling out of the locking portion.
(6) The blood purification device 1 defined as any one of (1) to (5), further comprising: a peristaltic pump 34 that applies pressure to a fluid in a pump tube 31a attached to the cassette frame 28, wherein the peristaltic pump 34 introduces the pump tube 31a into the interior thereof by being driven from a predetermined rotational position.
   It is thereby possible to attach the cassette frame easily.
(7) The blood purification device 1 as defined by (6), further comprising: a control unit 43 that controls the peristaltic pump 34 to execute a self-diagnostic action to diagnose the peristaltic pump 34 and a loading preparation action to rotate the peristaltic pump 34 to the predetermined rotational position, and an operation unit 42 to receive a self-diagnostic operation, wherein when receiving the self-diagnostic operation through the operation unit 42, the control unit 43 executes the loading preparation action in addition to the self-diagnostic action.

It is thereby possible to reduce the user's time and effort for operation.

### REFERENCE SIGNS LIST

1: blood purification device, 28: cassette frame, 31a: pump tube ,33: extracorporeal circulation circuit, 34: peristaltic pump, 41: cassette frame attachment part, 42: operating and display unit, 43: control unit, 51: right locking portion, 51b: locking protrusion, 51c: contact portion, 52, 53: left locking portion, 52b, 53b: locking protrusion, 52c, 53c: contact portion 54: contact-type sensor, 55: displacing portion, 96: locking hole, 97: pressure-receiving portion, 97a: pressure-receiving surface, P1: first locking portion position, P2: second locking portion position

## Claims

1. A blood purification device, comprising:
a cassette frame that, when a component of an extracorporeal circulation circuit is attached thereto, forms an integral cassette; and
a cassette frame attachment part to which the cassette frame is removably attached,
wherein the cassette frame attachment part comprises a locking portion that locks the cassette frame from the front surface side, and a pressing portion that presses the cassette frame, which is locked by the locking portion, from the back surface side,
wherein the cassette frame comprises a locking hole through which the locking portion passes, and a pressure-receiving portion that receives pressure from the pressing portion, and
wherein one of the pressing portion and the pressure-receiving portion comprises an inclined surface that comes into contact with the other of the pressing portion and pressure-receiving portion and converts a part of a pressing force from the pressing portion into a force in an opposite direction to a direction a locking protrusion of the locking portion faces, thereby moving the cassette frame in the opposite direction to the direction the locking protrusion faces.

2. The blood purification device according to claim 1, wherein the cassette frame attachment part comprises a plurality of the locking portions, wherein the locking protrusions of the plurality of locking portions face the same direction, and wherein the inclined surface converts a part of the pressing force from the pressing portion into a force in an opposite direction to the direction the locking protrusions of the plurality of locking portions face.

3. The blood purification device according to claim 1 or 2, wherein the cassette frame attachment part further comprises a displacing portion that displaces the locking portion between a first locking portion position, at which the cassette frame comes into contact with the pressing portion, and a second locking portion position, at which the cassette frame is separated from the pressing portion.

4. The blood purification device according to claim 1 or 2, wherein the pressing portion comprises a contact-type sensor that comes into contact with the pressure-receiving portion to detect attachment of the cassette frame.

5. The blood purification device according to claim 1 or 2, wherein the locking portion comprises a contact portion that comes into contact with the back surface side of the cassette frame.

6. The blood purification device 1 or 2, further comprising:
a peristaltic pump that applies pressure to a fluid in a pump tube attached to the cassette frame,
wherein the peristaltic pump introduces the pump tube into the interior thereof by being driven from a predetermined rotational position.

7. The blood purification device according to claim 6, further comprising:
a control unit that controls the peristaltic pump to execute a self-diagnostic action to diagnose the peristaltic pump and a loading preparation action to rotate the peristaltic pump to the predetermined rotational position, and
an operation unit to receive a self-diagnostic operation,
wherein when receiving the self-diagnostic operation through the operation unit, the control unit executes the loading preparation action in addition to the self-diagnostic action.
